(19) Europäisches Patentamt
European Patent Office
Office européen des brevets

(11) EP 0 741 130 B1

(12) **EUROPÄISCHE PATENTSCHRIFT**

(45) Veröffentlichungstag und Bekanntmachung des Hinweises auf die Patenterteilung:
**01.12.1999 Patentblatt 1999/48**

(21) Anmeldenummer: **95106730.5**

(22) Anmeldetag: **04.05.1995**

(51) Int Cl.6: **C07C 315/04**, C07C 317/28, C07C 303/24, C07C 305/06, C07C 315/02, C07C 317/18, C09B 62/503, C09B 62/51

(54) **Verfahren zur Herstellung von Sulfonen**

Method for the preparation of sulphones

Procédé pour la préparation des sulfones

(84) Benannte Vertragsstaaten:
**CH DE FR GB LI**

(43) Veröffentlichungstag der Anmeldung:
**06.11.1996 Patentblatt 1996/45**

(73) Patentinhaber: **BAYER AG**
**51368 Leverkusen (DE)**

(72) Erfinder:
- **Harms, Wolfgang, Dr.**
  **D-51519 Odenthal (DE)**
- **Hendricks, Udo-Winfried, Dr.**
  **D-51519 Odenthal (DE)**
- **Herd, Karl-Josef, Dr.**
  **D-51519 Odenthal (DE)**
- **Kunde, Klaus, Dr.**
  **D-53819 Neunkirchen (DE)**

(56) Entgegenhaltungen:
**EP-A- 0 568 876**
**EP-A- 0 627 667**
**DE-A- 4 331 163**
**GB-A- 1 156 172**
**US-A- 3 006 963**
**US-A- 3 094 455**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

**Beschreibung**

Gegenstand der vorliegenden Erfindung ist ein Verfahren zur Herstellung von Verbindungen der Formel

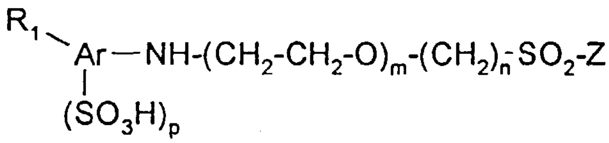

(1)

worin

Ar = Phenyl oder Naphthylrest,
$R_1$ = H, $C_1$-$C_4$-Alkyl, Cl, Br, $C_1$-$C_4$-Alkoxy oder COOH, Acylamino, insbesondere Acetamino, $SO_2$-Alkylen-OH,
m = 0 oder 1
n = 2, 3 oder 4,
p = 0-2,
Z = $-CH{=}CH_2$, $-CH_2-CH_2-OSO_3H$, $-CH_2-CH_2-S_2O_3H$, $-CH_2-CH_2-OCOCH_3$, $CH_2-CH_2-OPO_3H_2$, $-CH_2-CH_2-OH$,

dadurch gekennzeichnet, daß man in Sulfonen der Formel

$$X\text{-}(CH_2\text{-}CH_2\text{-}O)_m\text{-}(CH_2)_n\text{-}SO_2\text{-}CH_2\text{-}CH_2OH \qquad (2)$$

worin

X = Cl, Br, $OCO-(CH_2)_{0-3}-CH_3$, $O-CO-C_6H_5$,

den Rest X gegen

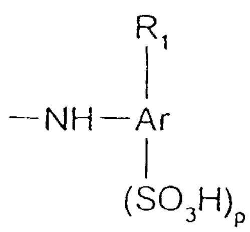

austauscht.

Ein alternatives Verfahren zur Herstellung von Verbindungen der Formel 1 ist aus EP-A 568 876 bekannt, dabei wird unter Einsatz von Mercaptoethanol zunächst ein Thioether gebildet, der anschließend zum Sulfon oxidiert wird.

Die so erhaltene Verbindung der Formel

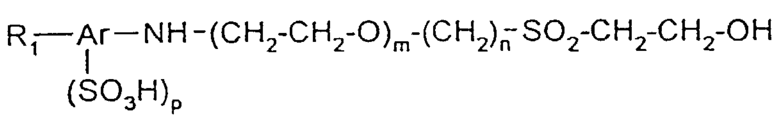

(5)

in der Z = $-C_2H_4-OH$, kann in üblicher Weise durch Funktionalisierung der OH-Gruppe in Verbindungen überführt werden, in denen Z eine der anderen angegebenen Bedeutungen hat.

In einer bevorzugten Ausführungsform führt man, falls p = 0 ist, gegebenenfalls noch $SO_3H$-Gruppen in den Aromatenrest ein.

Gegenstand der Erfindung sind auch Sulfone der Formel

$$X\text{-}(CH_2\text{-}CH_2\text{-}O)_q\text{-}(CH_2)_r\text{-}SO_2\text{-}CH_2\text{-}CH_2OH \quad (6)$$

worin

X = Cl, Br, $OCO\text{-}(CH_2)_{0\text{-}3}\text{-}CH_3$, $O\text{-}CO\text{-}C_6H_5$,
q = 0 oder 1 und
r = 2, 3 oder 4, wenn q = 1

oder

r = 3 oder 4, wenn q = 0

sowie das Verfahren zur Herstellung von Sulfonen der Formel (6), dadurch gekennzeichnet, daß man Thioether der Formel

$$X\text{-}(CH_2\text{-}CH_2\text{-}O)_q\text{-}(CH_2)_r\text{-}S\text{-}CH_2\text{-}CH_2\text{-}OH \quad (7)$$

worin

q und r die oben angegebene Bedeutung haben,

in wäßriger oder wäßrig-organischer Lösung vorzugsweise mit Wasserstoffperoxid unter Katalyse von Wolfram- oder Vanadiumverbindungen oxidiert.

Thioether der Formel (7) sind bekannt und mehrfach in der Literatur beschrieben:
Tsou et al. Journal of Organic Chemistry 26 (1961) 4987-89
Su et al. Journal of Organic Chemistry 26 (1961) 4990
Gilman, Tolman Journal Americ. Soc. 67 (1945) 1848
D'Agostino, Porter Rapid Commun. Mass. Spectrom. 6 (1992) 717.

Sie können beispielsweise durch halbseitige Umsetzungen von Dihalogenverbindungen der Formel

$$X\text{-}(CH_2\text{-}CH_2\text{-}O)_m\text{-}(CH_2)_n\text{-}Y \quad (8)$$

worin

X, Y = Cl oder Br

und m und n die oben angegebene Bedeutung haben,
mit Mercaptoethanol oder durch zweckmäßigerweise radikalkatalysierte Addition (siehe z.B. Organic Reactions Bd. 13, Seite 179 und 189) von Mercaptoethanol an Vinylverbindungen wie beispielsweise Vinylacetat hergestellt werden.

Beschrieben in der Literatur sind auch die Sulfone der Formel (9) US-Patentschrift 3 509 218 der Formel (10) ebenda und Schöberl, Biedermann Liebigs Ann. Chemie 716 (1968) 37-46

$$Cl\text{-}CH_2\text{-}CH_2\text{-}SO_2\text{-}CH_2\text{-}CH_2OH \quad (9)$$

$$CH_2{=}CH\text{-}SO_2\text{-}CH_2\text{-}CH_2OH \quad (10).$$

Der Austausch des Restes X in den Sulfonen der Formel (2) gegen einen Rest, insbesondere einen Anilinrest

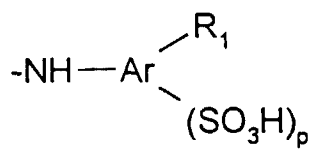

kann in wäßrigem, wäßrig-organischem oder rein organischem Medium entweder unter kontrollierten pH-Bedingungen oder in Gegenwart basischer Mittel erfolgen, wie z.B. Alkalicarbonat oder -hydrogencarbonat, Alkaliphosphate, Alkalihydroxid, Calciumoxid, Magnesiumoxid.

Kontrollierte pH-Bedingungen sind pH-Werte zwischen 5 und 12. Der optimale pH-Wert hängt von der Struktur des Sulfons (2) ab.

Für den Austausch einsetzbare Lösungsmittel sind $C_1$-$C_5$-Alkohole, Aceton, Methylethylketon, N-Methyl-pyrrolidon, Dimethylformamid, Dimethylsulfoxid, Tetramethylensulfon.

Im Prinzip ist es möglich, sowohl in wäßrigem als auch in wäßrig organischem Medium die Oxidation des Thioethers zu den Sulfonen (2) und den Austausch des Substituenten X in den Sulfonen zu den Verbindungen der Formel (1) in einem Zuge vorzunehmen, ohne daß eine Zwischenisolierung des Sulfons (2) erforderlich ist.

Die nach dem beschriebenen Verfahren hergestellten Verbindungen der Formel (1) sind wertvolle Zwischenprodukte für die Synthese von Reaktivfarbstoffen, die insbesondere zum Färben von natürlichen und regenerierten Cellulosefasern eingesetzt werden.

Die Verbindungen der Formel (1) können für die Synthese derartiger Farbstoffe (4a), beispielsweise mit Diazoniumsalzen als Kupplungskomponenten, eingesetzt werden,

$$D\text{-}N{=}N\text{-}Ar\text{-}NH\text{-}(CH_2\text{-}CH_2\text{-}O)_m\text{-}(CH_2)_n\text{-}SO_2\text{-}CH_2\text{-}CH_2\text{-}OSO_3H \qquad (4a)$$

worin

D den Rest einer Diazokomponente bedeutet, und

m und n die oben angegebene Bedeutung haben.

Bevorzugt dienen Verbindungen der Formel (1) jedoch durch Kondensation mit Trihalogentriazinen und aminogruppenhaltigen Farbbasen zum Aufbau hochfixierender bifunktioneller Reaktivfarbstoffe der Formel

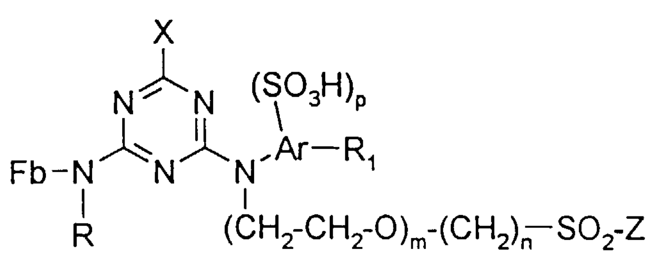

(4)

worin

Z, $R_1$, p, m und n die zu Verbindung (1) angegebene Bedeutung haben und

X = F, Cl, Br.

R = H oder $C_1$-$C_4$-Alkyl,

Fb = der Rest eines Farbstoffes der Mono- oder Polyazo-, Metallkomplexazo-, Anthrachinon-, Phthalocyanin-, Formazan-, Azomethin-, Dioxazin-, Phenazin-, Stilben-, Triphenylmethan-, Xanthen-, Thioxanthon-, Nitroaryl-, Naphthochinon-, Pyrenchinon- oder Perylentetracarbimid-Reihe.

**Beispiel 1**

A. 129 g Vinylacetat und 140,4 g 2-Mercaptoethanol werden gemischt. Man setzt unter Rühren 1,5 g Azo-bis-(isobutyronitril) hinzu und rührt das Gemisch weiter, wobei die Temperatur in ca. 30 Minuten auf 30°C ansteigt. Man rührt die Mischung über Nacht bei Raumtemperatur und erwärmt sie anschließend 8 Stunden auf 50°C, bis eine in DMSO gelöste Probe keine für Vinylacetat charakteristischen $^1H$-NMR-Signale bei 7,2 ppm (Vierfachaufspaltung des einzelnen CH-Vinylprotons) und bei 4,6 ppm und um 4,85 ppm der $CH_2$-Vinylprotonen (jeweils Doppelaufspaltung) mehr zeigt.

Es liegt ein farbloses Öl vor, dessen Hauptbestandteil der Formel

$$CH_3\text{-}CO\text{-}O\text{-}CH_2\text{-}CH_2\text{-}S\text{-}CH_2\text{-}CH_2\text{-}OH$$

entspricht.
$^1H$-NMR in $D_6$-DMSO (TMS als innerer Standard)

δ = 2,00 ppm (s, 3H)
= 2,60 ppm (t, 2H)
= 2,76 ppm (t, 2H)
= 3,55 ppm (m, 2H)
= 4,13 ppm (t, 2H)
= 4,79 ppm (t, 1H)

B. 81,3 g Thioether in Form des Öles aus Beispiel 1A werden mit 225 ml Wasser und 5,25 g Na-acetat verrührt; man stellt mit ca. 1,5 ml Eisessig den pH-Wert auf 5,2 ein und versetzt mit einer Katalysatorlösung, die man aus 1,5 g Wolframsäure durch Neutralisation mit Natronlauge in 30 ml Wasser und Einstellen des pH-Wertes mit ca. 2,4 ml Eisessig auf pH 5,2 hergestellt hat.

90,0 g 35 %iges Wasserstoffperoxid werden bei 55°C zugetropft, wobei der pH-Wert mit 2n Natronlauge auf 5,0-5,2 gehalten wird. Man setzt noch soviel 35 %iges Wasserstoffperoxid nach, wie zur Aufrechterhaltung eines schwachen Überschusses nötig ist. Hierfür werden in der Regel nochmals 20-30 g benötigt. Wenn nach 4 Stunden bei 55-60°C das gesamte Sulfoxid zum Sulfon oxidiert ist, was durch eine dünnschichtchromatographische Probe feststellbar ist, kühlt man auf 35°C ab. Man erhält eine farblose Lösung.

Engt man die erhaltene Lösung bei 35-40°C im Vakuum ein, so erhält man die Substanz

$$CH_3\text{-}CO\text{-}O\text{-}CH_2\text{-}CH_2\text{-}SO_2\text{-}CH_2\text{-}CH_2OH$$

in Form eines farblosen Öls, das im Massenspektrum mit chemischer Ionisation folgende Massen liefert:
$M_1 + H^+ = 197$
$M_2 + = 155$ (Abspaltung $CH_3$-CO)
$M_3 + H^+ = 137$ (Abspaltung $H_2O$)
$^1H$-NMR in $D_6$-DMSO (TMS als innerer Standard)

δ = 2,02 ppm (s, 3H)
= 2,66 ppm (t, 2H)
= 3,51 ppm (t, 2H)
= 3,65 ppm (t, 2H)
= 4,38 ppm (t, 2H)
≈ 4,6 ppm (breit)

C. Die im Beispiel 1B erhaltene farblose Lösung des 2-Acetoxyethyl-2'-hydroxyethylsulfons (0,45 Mol) wird bei 50°C mit 84 g Anilin versetzt. Man stellt den pH-Wert mit 2n Natronlauge auf 6,5 und rührt die Mischung bei 55°C und pH 6,5 mehrere Stunden, bis eine chromatographische Probe das Verschwinden des Acetoxysulfons anzeigt. Man treibt nun überschüssiges Anilin bis zum Verschwinden mit dem überdestillierenden Wasser im Vakuum bei 50-60°C ab und erhält nach Abtrennung ein bräunliches, allmählich zu einer wachsähnlichen Masse erstarrendes Öl.

Ausbeute: 186 g mit einem Gehalt von 34 % (HPLC) der Verbindung der Formel

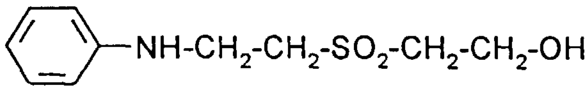

(Ausbeute von 61 % d.Th. bezüglich des im Beispiel 1A eingesetzten Vinylacetats).

Das isolierte und scharf getrocknete Produkt zeigt folgende physikalische Eigenschaften:
Schmelzpunkt nach scharfer Trocknung <50°C.
Massenspektrum mit chemischer Ionisation $M_1 + H^{\oplus} = 230$
$^1$H-NMR in $D_6$-DMSO (TMS als innerer Standard)

δ = 3,27-3,38 (m, 4H)
= 3,48-3,53 (m, 2H)
= 3,74-3,82 (m, 2H)
= 5,23 (t, 1H)
= 5,77 (t, 1H)
= 6,55-6,60 (m, 3H)
= 7,05-7,12 (m, 2H)

Als Nebenbestandteil enthält das Rohprodukt neben 34 % Mol 229 obiger Formel noch 2-3 % Bis-(2-hydroxyethyl)-sulfon.

**Beispiel 2**

Die im Beispiel 1B erhaltene farblose Lösung des 2-Acetoxyethyl-2'-hydroxyethylsulfons (0,45 Mol) wird mit 44 g Anilin versetzt, die Mischung auf pH 7,0 gestellt und zunächst 4 Stunden bei 50°C und später bei 60°C bis zum Verschwinden des Acetoxysulfons verrührt. Nach Eindampfen der Mischung im Vakuum erhält man das in Beispiel 1C beschriebene Produkt mit ähnlichem Gehalt.

**Beispiel 3**

Die im Beispiel 1B erhaltene farblose Lösung des 2-Acetoxyethyl-2'-hydroxyethylsulfons (0,45 Mole) wird mit 44 g Anilin versetzt und bei pH 6,5 unter Zudosierung des notwendigen Bedarfs an 2n Natronlauge etwa 12 Stunden auf 50-55°C gehalten, bis eine dünnschichtchromatographische Probe oder HPLC-Probe praktisch kein Acetoxysulfon mehr zeigt. Nach Eindampfen der Mischung im Vakuum erhält man das im Beispiel 1C beschriebene N-(2-(2'-Hydroxyethylsulfonyl)-ethyl)-anilin in ähnlicher Form und Qualität.

**Beispiel 4**

Man wiederholt den Versuch des Beispiels 3 mit 66 g Anilin-Einsatz, arbeitet im übrigen entsprechend und treibt zur Aufarbeitung das überschüssige Anilin durch Vakuumdestillation mit Wasserdampf ab, wonach man ein dem Beispiel 1C entsprechendes Produkt erhält.

**Beispiel 5**

Man wiederholt den Versuch des Beispiels 4, hält jedoch den pH-Wert zwecks Austausches der Acetoxygruppe gegen den Anilinrest bei 5,5.

**Beispiel 6**

Man wiederholt den Versuch des Beispiels 4, hält jedoch den pH-Wert zwecks Austausches der Acetoxy-Gruppe gegen den Anilinrest bei 7,5-8,0.

Weitere Produkte der allgemeinen Formel

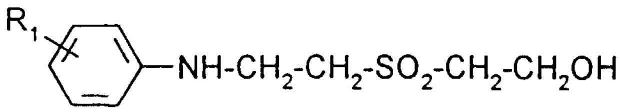

erhält man, wenn man in den Beispielen 2 bis 7 anstelle des Anilins folgende substituierte Aniline einsetzt:

| Beispiel-Nr. | Substituiertes Anilin |
|---|---|
| 7 | o-Toluidin |
| 8 | p-Toluidin |
| 9 | m-Toluidin |
| 10 | p-Anisidin |
| 11 | m-Anisidin |
| 12 | o-Anisidin |
| 13 | p-Chloranilin |
| 14 | 4-Acetaminoanilin |
| 15 | 3-Acetaminoanilin |
| 16 | 3-Aminobenzolsulfonsäure |
| 17 | 4-Aminobenzolsulfonsäure |
| 18 | 2-Aminonaphthalin-5-sulfonsäure |
| 19 | 3-Aminophenyl-2'-hydroxyethylsulfon |
| 20 | 3-Aminobenzoesäure |
| 21 | 4-Aminobenzoesäure |
| 22 | 2-Aminobenzoesäure |

**Beispiel 23**

80,0 g des nach Beispiel 2-5 erhaltenen Sulfons der Formel

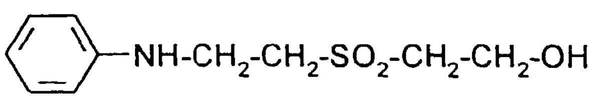

werden in 40 ml 96 %ige Schwefelsäure bei 20-30°C eingetragen und 3 Stunden verrührt. Man tropft unter Kühlung bei 10-15°C 50 ml 20 %iges Oleum zu und rührt 2-3 Stunden nach, bis im Dünnschichtchromatogramm praktisch kein Edukt mehr nachweisbar ist. Die erhaltene Lösung wird auf 900 g Eiswasser gegeben. In die verdünnt schwefelsaure Suspension werden nun bei 0-10°C 210-220 g Calciumcarbonat eingetragen, bis ein pH-Wert von 5,0 erreicht ist.

Nach Filtration vom Calciumsulfat, Waschen des Niederschlages und Einengen der vereinigten Filtrate auf ein gewünschtes Volumen erhält man die Lösung der Verbindung

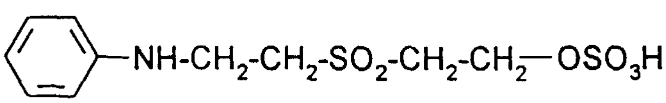

in einer Ausbeute von 94 % (Titration mit Natriumnitrit).

**Beispiel 24**

Wiederholt man den Ansatz des Beispiels 23 mit gleichen Mengen N-(2-(2-Hydroxyethylsulfonyl)-ethyl)-anilin, 96 %iger Schwefelsäure und 20 %igem Oleum in gleicher Weise und trägt die erhaltene Lösung am Schluß unter Kühlung in 500 g Eis ein, rührt 1 Stunde bei 0-5°C nach, so erhält man eine Fällung des Produktes der Formel

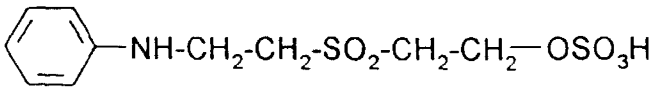

in Form eines hellbraunen Niederschlages mit einer Ausbeute von 55-65 %, der durch Anschlagen in Isopropanol, Absaugen und Waschen mit Isopropanol von Schwefelsäureresten befreit werden kann.
$^1$H-NMR in $D_6$-DMSO (TMS als innerer Standard)

δ = 3,43 ppm (2H, t)
δ = 3,50 ppm (2H, t)
δ = 3,60 ppm (2H, t)
δ = 4,11 ppm (2H, t)
δ = 7,00 ppm (3H, d, d)
6 = 7,30 ppm (2H, t)
δ = 8,98 ppm (2H, s)

**Beispiel 25**

A. 81,3 g 2-Acetoxyethyl-2'-hydroxyethylsulfid aus Beispiel 1A werden in 150 ml N-Methyl-2-p-pyrrolidon gelöst. Man gibt eine Lösung von 2,0 g Natriumwolframat ($Na_2WO_4.2H_2O$) in 10 ml Wasser hinzu, die man mit Eisessig auf pH 5,5 gestellt hat, und läßt unter Kühlung bei 50°C 90,0 g 35 %iges Wasserstoffperoxid zulaufen.

Die Temperatur wird bei 50°C und der pH-Wert mit 2n-Natronlauge auf 5,0-5,2 gehalten.

Diese Bedingungen hält man weiter ein und setzt nach 2 Stunden gegebenenfalls noch die erforderliche Menge Wasserstoffperoxid nach zwecks vollständiger Oxidation zum 2-Acetyloxyethyl-2'-hydroxyethylsulfon.

B. Nach beendeter Oxidation läßt man 44 g Anilin zulaufen. Der pH-Wert wird nun auf 6,5 gehalten und die Mischung mehrere Stunden bei 100°C bis zum Verschwinden der Acetoxyethylverbindung gerührt. Man erhält eine Lösung des Produktes der Formel

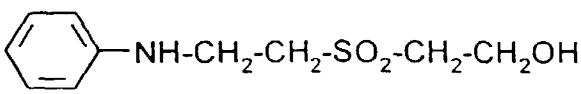

Aus der erhaltenen Lösung wird das Wasser im Vakuum (1 mm) abdestilliert.

C. Läßt man zu der nach Beispiel 25B erhaltenen N-Methylpyrrolidon-Lösung von N-(2-(2'-Hydroxyethylsulfonyl)-ethyl)-anilin 70 g Chlorsulfonsäure zulaufen, kontrolliert auf Verschwinden des Eduktes, gibt die erhaltene Lösung auf 300 g Eis unter Kühlung und neutralisiert sie bei 0-5°C mit festem Lithiumcarbonat oder 15 %iger Sodalösung, so erhält man eine Lösung der Verbindung

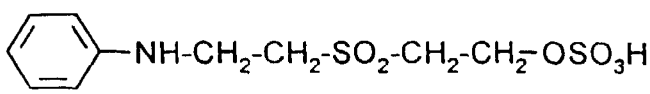

die direkt weiter verarbeitet werden kann.

**Beispiel 26**

45,8 g N-(2-(2-Hydroxyethylsulfonyl)-ethyl)-anilin werden bei 20-25°C in eine Mischung von 120 ml 20 %igem und 35 ml 65 %igem Oleum eingetragen. Man erwärmt die Lösung auf 40°C. Wenn eine Probe weder Edukt, noch die in den Beispielen 23, 24 und 25 beschriebene Sulfatoverbindung mehr anzeigt, wird die Reaktionslösung auf 900 g Eis und 300 ml Wasser ausgetragen.

Die erhaltene braune Lösung wird mit ca. 325 g Calciumcarbonat bei 0-10°C bis zum pH-Wert von 5,5 neutralisiert. Man filtriert vom ausgefälltem Calciumsulfat ab, wäscht dieses mit Wasser frei vom Produkt und erhält nach Einengen eine braune Lösung der Verbindung der Formel (isomere Sulfonsäuren m:p = 70:30)

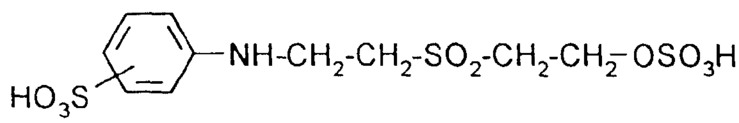

in einer Ausbeute von 94 % (Titration mit Natriumnitrit-HCl).

Engt man die Lösung zur Trockene ein, so verbleibt eine braune, zähe Masse, die sich durch Verreiben mit Isopropanol in ein sandfarbenes Pulver umwandeln läßt. Trocknung im Umluftschrank.

$^1$H-NMR in $D_6$-DMSO (TMS als innerer Standard)

| | Signalintegration |
|---|---|
| δ = 3,35 ppm (m, 2H)<br>δ = 3,42-3,58 ppm (m, 4H)<br>δ = 4,10-4,16 ppm (m, 2H) | 8 H |
| δ = 6,58 ppm (d, 2H, p-Isomeres)<br>δ = 7,40 ppm (d, 2H, p-Isomeres)<br>δ = 6,63-6,69 ppm (d, d, 1H, m-Isomeres)<br>δ = 6,90 ppm (s, 1H, m-Isomeres)<br>δ = 6,95 ppm (d, 1H, m-Isomeres)<br>δ = 7,13-7,20 ppm (t, 1H, m-Isomeres) | 4 H |

**Beispiel 27**

Die im Beispiel 1B erhaltene Lösung des 2-Acetoxyethyl-2'-hydroxyethylsulfons (0,45 Mole) wird mit 2n Natronlauge bei Raumtemperatur mehrere Stunden auf pH 10 gehalten, bis der Natronlaugeverbrauch zum Stillstand kommt und die Bildung des 2-Hydroxyethyl-vinyl-sulfons beendet ist. Man setzt 44 g Anilin zu der Lösung und erwärmt 6 Stunden auf 50°C, bis die Anilinaddition vollständig ist. Nach anschließendem Eindampfen im Vakuum verbleibt ein Öl, dessen Hauptbestandteil aus N-(2-(2'-Hydroxy-ethylsulfonyl)-ethyl)-anilin besteht.

**Beispiel 28**

54,4 g 2-Hydroxyethyl-vinylsulfon, hergestellt nach A. Schöberl, M. Biedermann, Liebigs Ann. Chemie 716, 37-46 (1968), werden in 400 ml Wasser verrührt. Nach Zusatz von 45 g Anilin wird der pH-Wert auf 8-9 eingestellt und die Mischung mehrere Stunden auf 50-55°C erwärmt, bis die Vinylverbindung vollständig umgesetzt ist. Nach Entfernen

des überschüssigen Anilins mit Wasserdampf im Vakuum erhält man als Rückstand ein langsam erstarrendes Öl des N-(2-(2'-Hydroxyethylsulfonyl)-ethylanilins mit den im Beispiel 2 gegebenen physikalischen Daten.

## Beispiel 29

60,0 g 3-Chlorpropyl-2'-hydroxyethylsulfid, hergestellt durch Umsetzung von 1,3-Bromchlorpropan mit 2-Mercaptoethanol (vgl. Journal Americ. Soc. 67 (1945) 1848) werden in 180 ml Wasser und 4,5 g Natriumacetat verrührt. Man gibt eine Lösung von 1,2 g Wolframsäure in 30 ml Wasser und 1,8 ml 50 %iger Natronlauge hinzu und stellt dann den pH-Wert mit 3,0 ml Eisessig auf 5,2. Man läßt nun bei 25-30°C in 40 Minuten 38,7 g 35 %iges Wasserstoffperoxid zutropfen, wobei die zu Beginn vorhandene Emulsion sich in eine klare Lösung verwandelt. Erneut werden weitere 38,7 g 35 %iges Wasserstoffperoxid zugetropft, wobei man die Temperatur auf 45-50°C steigert und den pH-Wert durch Zutropfen von etwa 6-7 ml 2n Sodalösung auf 5,2 hält. Der Ansatz wird noch 1-1½ Stunden auf 50°C bei positiver Probe auf $H_2O_2$ gehalten, bis eine chromatographische Probe kein Sulfoxid mehr anzeigt.

Die erhaltene Lösung kann zwecks Austausch von Chlor in diesem Zustand weiter verarbeitet werden. Will man das Produkt isolieren, so dampft man die Reaktionslösung im Rotationsverdampfer im Vakuum ein, nimmt den Rückstand in 300 ml Methylenchlorid auf, saugt von Salzen ab, wäscht sie mit Methylenchlorid nach und dampft die vereinigten Filtrate ein. Die Verbindung der Formel

$$Cl\text{-}(CH_2)_3\text{-}SO_2\text{-}CH_2\text{-}CH_2\text{-}OH$$

wird als helles, gelbes Öl erhalten mit einer Ausbeute von 70,2 g = 97% der Theorie.
$^1$H-NMR in $D_6$-DMSO (TMS als innerer Standard)

$\delta$ = 2,12-2,24 (m, 2H)
$\delta$ = 3,23-3,28 (m, 4H)
$\delta$ = 3,73-3,381 (m, 4H)
$\delta$ = ca. 5,2 (breit, 1H)

## Beispiel 30

Die in Beispiel 29 erhaltene Lösung von 70,2 g 3-Chlorpropyl-2'-hydroxyethylsulfon oder eine Lösung von 70,2 g des isolierten Sulfons in 280 ml Wasser wird mit 37,9 g Anilin und 34,4 g Natriumhydrogencarbonat versetzt. Die Emulsion wird 12 Stunden auf 100°C erhitzt, bis eine chromatographische Probe den vollständigen Austausch des Chloratoms gegen den Anilinrest anzeigt. Man treibt überschüssiges Anilin durch Wasserdampf im Vakuum ab und erhält nach Absetzenlassen ein braunes Öl, das abgetrennt wird. Nach Extraktion der wäßrigen Phase mit 200 ml Methylenchlorid werden abgetrenntes Öl und Methylenchlorid-Phase vereinigt, mit 150 ml Wasser gewaschen und über Natriumsulfat getrocknet. Nach Eindampfen der Methylenchloridlösung, zuletzt im Vakuum, bleiben als Rückstand 68,0 g = 74 % der Theorie eines hellbraunen Öls der Formel

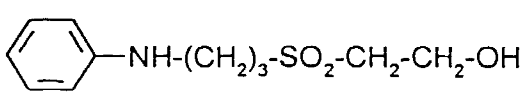

Massenspektrum Molmasse $M^+$ = 243
$^1$H-NMR in $D_6$-DMSO (TMS als innerer Standard)

$\delta$ = 1,92-2,03 (m, 2H)
$\delta$ = 3,09-3,24 (m, 6H)
$\delta$ = 3,79-3,85 (m, 2H)
$\delta$ = 5,08-5,12 (t, 1H)
$\delta$ = 5,58-5,62 (t, 1H)
$\delta$ = 6,48-6,58 (m, 3H)
$\delta$ = 7,04-7,10 (t, 2H)

**Beispiel 31**

64,8 g N-3-(2-Hydroxyethylsulfonyl)-propylanilin aus Beispiel 30 werden in eine Mischung von 54 ml 96 %iger Schwefelsäure und 54 ml 20 %igem Oleum in einer Stunde eingetropft. Man hält im Reaktionsgemisch die Temperatur durch Kühlung auf 20-25°C. Wenn nach zwei- bis dreistündigem Nachrühren eine chromatographische Probe praktisch kein Edukt mehr anzeigt, wird der Ansatz auf 600 g Eis gegossen. Man neutralisiert die Lösung in etwa eineinhalb Stunden durch Zusatz von ca. 309 g Calciumcarbonat bis zum pH 5,0, saugt das gefällte Calciumsulfat ab, wäscht den Gips mit ca. 2 1 Wasser und engt Filtrat und Wäsche im Vakuum bei 12-15 Torr ein bis auf ein Restvolumen von ca. 600 ml.

Die erhaltene Lösung der Verbindung

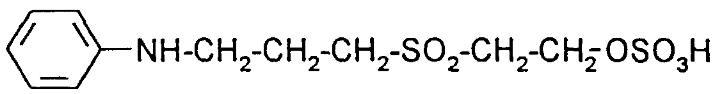

deren Gehalt man durch Titration mit Natriumnitritlösung ermittelt, kann direkt zur Herstellung von Reaktivfarbstoffen, beispielsweise durch Kondensation mit Cyanurfluorid oder Cyanurchlorid und anschließende Umsetzung des Kondensationsproduktes mit einer aminogruppenhaltigen Farbbase, eingesetzt werden.

Will man den Schwefelsäurehalbester in Form eines Salzes isolieren, so dampft man die nach der Gipsfällung erhaltene Lösung nach nochmaliger, zwischenzeitlicher Filtration ein, verrührt den Eindampfrückstand mit Isopropanol, saugt ab und trocknet.

**Beispiel 32-45**

Weitere Produkte der allgemeinen Formel

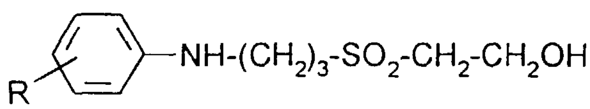

erhält man, wenn man im Beispiel 30 anstelle des Anilins folgende substituierte Aniline einsetzt:

| Beispiel-Nr. | substituiertes Anilin |
|---|---|
| 32 | p-Toluidin |
| 33 | m-Toluidin |
| 34 | o-Toluidin |
| 35 | p-Anisidin |
| 36 | m-Anisidin |
| 37 | o-Anisidin |
| 38 | 3-Aminobenzolsulfonsäure |
| 39 | 3-Aminophenyl-2'-hydroxyethylsulfon |
| 40 | 4-Aminophenyl-2'-hydroxyethylsulfon |
| 41 | 4-Acetaminoanilin |
| 42 | 3-Acetaminoanilin |
| 43 | 3-Aminobenzoesäure |
| 44 | 4-Aminobenzoesäure |
| 45 | 3-Nitroanilin |

**Beispiel 46**

33,7 g 4-Chlor-1-butyl-2'-hydroxyethylsulfid, hergestellt durch Umsetzung von 1,4-Dichlorbutan mit 2-Mercaptoethanol (analog Tsou et al., J. Org. Chem. 26 (1961), 4989) werden in 150 ml Wasser und 2,3 g Natriumacetat verrührt. Nach Zusatz von 0,6 g Wolframsäure in 15 ml Wasser und 0,9 ml 50 %iger Natronlauge oxidiert man den Thioether in der im Beispiel 29 beschriebenen Weise mit 41 g 35 %igem Wasserperoxid bei zuletzt 45-50°C und erhält eine Lösung des Sulfons der Formel

$$Cl\text{-}(CH_2)_4\text{-}SO_2\text{-}CH_2\text{-}CH_2\text{-}OH$$

das analog dem Beispiel 30 nach Zusatz von 20,0 g Anilin und 17 g Natriumhydrogencarbonat bei 100°C zur Verbindung der Formel

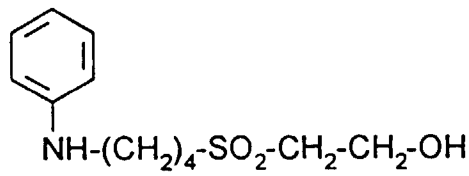

umgesetzt wird, die als hellbraunes Öl in der im Beispiel 30 gezeigten Weise isoliert werden kann.

**Beispiel 47**

36,9 g 2-(2-Chlorethoxy)-ethyl-2'-hydroxyethylsulfid
$^1$H-NMR in $D_6$-DMSO (TMS als innerer Standard)

δ = 2,56 - 2,62 (t, 2H)
δ = 2,68 - 2,74 (t, 2H)
δ = 3,48 - 3,61 (t, t 4H)
δ = 3,66 - 3,73 (m, 4H)
δ = 4,74 (t, 1H)

hergestellt durch Umsetzung von 2-(Bis-chlorethyl)-ether mit Mercaptoethanol, werden in 150 ml Wasser und 2,3 g Natriumacetat verrührt und nach Zusatz einer Lösung von 0,6 g Wolframsäure in 15 ml Wasser und 0,9 ml 50 %iger Natronlauge mit 42 g 35 %igem Wasserstoffperoxid mit stufenweiser Temperaturführung zunächst unter Kühlung bei 30°C und im zweiten Abschnitt bei 55-60°C oxydiert. Man erhält eine Lösung, aus der sich nach Eindampfen im Vakuum das Sulfon der Formel

$$Cl\text{-}CH_2\text{-}CH_2\text{-}O\text{-}CH_2\text{-}CH_2\text{-}SO_2\text{-}CH_2\text{-}CH_2\text{-}OH$$

als farbloses Öl abscheidet.
Massenspektrum Chemische Ionisation $M+H^+$ = 217/219
$^1$H-NMR in $D_6$-DMSO (TMS als innerer Standard)

δ = 3,25 - 3,30 (t, 2H)
δ = 3,38 - 3,43 ( t, 2H)
δ = 3,69 - 3,74 (m, 4H)
δ = 3,78 - 3,86 (m, 4H)
δ = 4,64 (breit, 1H)

Unterläßt man das Eindampfen der wäßrigen Lösung des obigen Sulfons, setzt 20,0 g Anilin und 17 Natriumhydrogencarbonat zu, erhitzt auf 100°C und verfährt im übrigen nach den Angaben des Beispiels 30, so erhält man die Verbindung der Formel

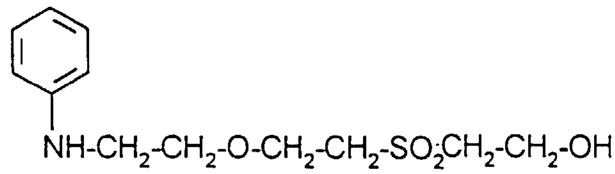

die als bräunliches Öl in der im Beispiel 30 gezeigten Weise isoliert werden kann.
Massenspektrum Chemische Ionisation
$M_1+H^+ = 274$  $M_1+H^+-H_2O = 256$

**Beispiel 48**

A)0,15 Mol der Verbindung der Formel

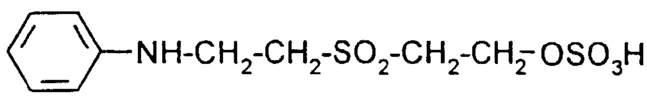

werden mit 100 Teilen Wasser und 100 Teilen Eis vermischt und mit $Na_2CO_3$ neutral gestellt. Zu dieser Lösung werden in 10 min bei 0°C 0,17 Mol 2,4,6-Trifluor-1,3,5-triazin zugetropft und der pH bei 4,5 bis 5 gehalten. Man erhält 250 Teile einer Kondensationslösung folgender Verbindung

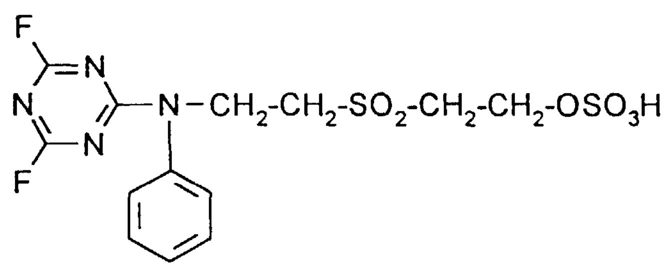

B) 0,1 Mole des Kupferkomplexes von N-(2-Carboxy-5-sulfophenyl)-N'-(2'-hydroxy-3'-amino-5'-sulfophenyl)-ms-phenyl-formazan-di-natriumsalz werden in 600 ml Wasser gelöst. Nach Abkühlung auf 0 bis 5°C läßt man die Lösung der in Beispiel 48A hergestellten Komponente zulaufen und hält den pH-Wert durch Zugabe von Sodalösung auf 7,0 bis 8,0. Nach 2 Stunden läßt man die Temperatur allmählich auf 20°C ansteigen und salzt nach beendeter Kondensation den Farbstoff aus, saugt ab und trocknet ihn nach Pufferung bei pH 6 bei 45°C im Vakuum.

Der Farbstoff der Formel

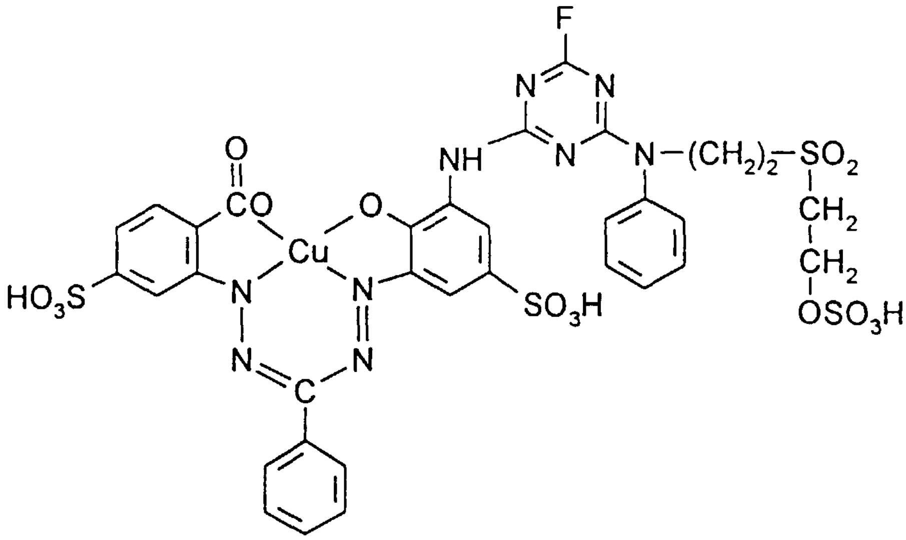

$\lambda_{max}$ = 609 nm ($H_2O$)
färbt Baumwolle aus langer Flotte mit sehr guten Fixierausbeuten in blauen Tönen.

**Beispiel 49**

A) 11,0 g Cyanurchlorid werden in 100 ml Eiswasser suspendiert. Man gibt 0,5 ml eines Emulgators hinzu. Eine Lösung von 56 mmolen der Komponente aus Beispiel 31 der Formel

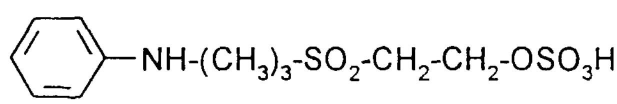

in 300 ml Wasser wird bei 0-5°C zugetropft und dabei der pH-Wert mit 2n Sodalösung auf 4,2-4,5 gehalten. Nach Klärung von geringen Mengen Cyanurchlorid-Resten wird die Lösung des erhaltenen Kondensationsproduktes zu einer neutralen Lösung von 0,45 Molen Monoazoverbindung der Formel

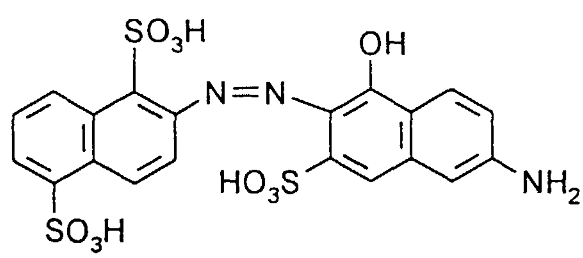

in 300 ml Wasser gegeben. Der Ansatz wird mit 2n Sodalösung auf pH 6,5 gehalten. Man hält die Temperatur zunächst 2 Stunden bei 30°C, steigert sie einige Stunden auf 40°C und zum Schluß auf 50°C, bis die Reaktionslösung kaum noch Monoazo-Edukt enthält. Aus der Lösung wird der Farbstoff mit 15 Gew.-% Natriumchlorid ausgesalzen, abgesaugt, mit Natriumchloridlösung gewaschen und nach Pufferung bei pH = 6 bei 45°C im Vakuum getrocknet. Man erhält ein orangefarbenes Pulver.

Der Farbstoff entspricht der Formel

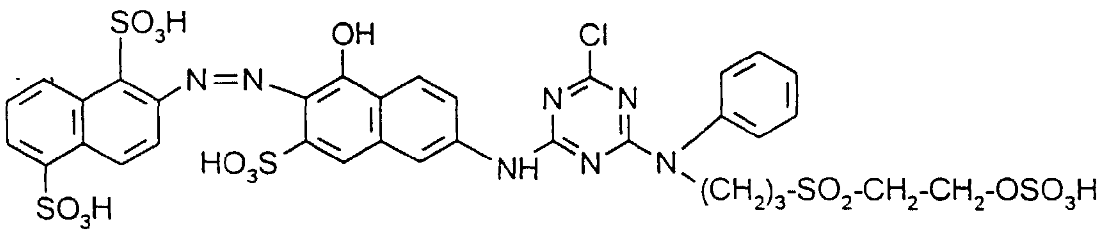

$\lambda_{max}$ = 486 nm in Wasser.
Er färbt Baumwolle aus langer Flotte mit hoher Fixierausbeute.

**Patentansprüche**

1. Verfahren zur Herstellung von Verbindungen der Formel

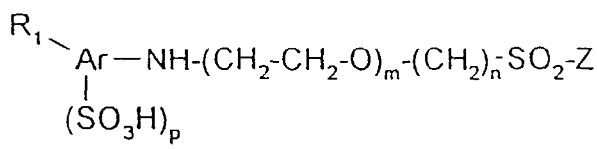
(1)

wonn

Ar = Phenyl oder Naphthylrest,
$R_1$ = H, $C_1$-$C_4$-Alkyl, Cl, Br, $C_1$-$C_4$-Alkoxy oder COOH, Acylamino, insbesondere Acetamino, $SO_2$-Alkylen-OH,
m = 0 oder 1
n = 2, 3 oder 4,
p = 0-2,
Z = $-CH{=}CH_2$, $-CH_2-CH_2-OSO_3H$, $-CH_2-CH_2-S_2O_3H$, $-CH_2-CH_2-OCOCH_3$, $CH_2-CH_2-OPO_3H_2$, $-CH_2-CH_2-OH$,

dadurch gekennzeichnet, daß man in Sulfonen der Formel

$$X-(CH_2-CH_2-O)_m-(CH_2)_n-SO_2-CH_2-CH_2OH \quad (2)$$

wonn

X = Cl, Br, $OCO-(CH_2)_{0-3}-CH_3$, $O-CO-C_6H_5$,

den Rest X gegen

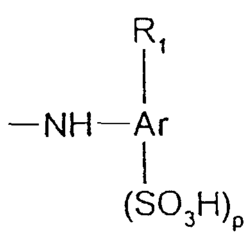

austauscht.

**2.** Verfahren nach Anspruch 1, dadurch gekennzeichnet, daß man eine Verbindung der Formel

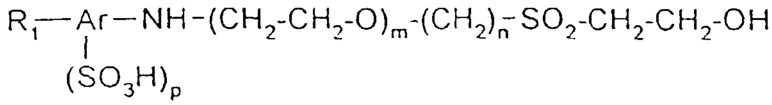

(5)

worin
$R_1$, p, m und n die in Anspruch 1 angegebene Bedeutung haben, in üblicher Weise durch eine Funktionalisierung der OH-Gruppe in Verbindungen überführt, in denen Z = $-CH=CH_2$, $-CH_2-CH_2-OSO_3H$, $-CH_2-CH_2-S_2O_3H$, $-CH_2-CH_2-OCOCH_3$, $CH_2-CH_2-OPO_3H_2$, sowie, falls p = 0 ist, gegebenenfalls noch $SO_3H$-Gruppen in den Aromatenrest einführt.

**3.** Verfahren gemäß Anspruch 1, dadurch gekennzeichnet, daß als Sulfon der Formel (2) 3-Chlorpropyl-2'-hydroxyethylsulfon mit Anilin zu einer Verbindung der Formel

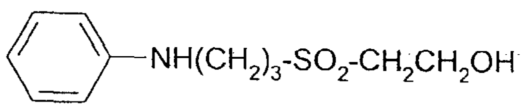

umgesetzt wird.

**4.** Sulfone der Formel

$$X-(CH_2-CH_2-O)_q-(CH_2)_r-SO_2-CH_2-CH_2-OH \quad (6)$$

worin

X = Cl, Br, $OCO-(CH_2)_{0-3}-CH_3$, $O-CO-C_6H_5$,
q = 0 oder 1,
r = 2, 3 oder 4, wenn q = 1,
r = 3 oder 4, wenn q = 0.

**5.** Verfahren zur Herstellung von Sulfonen gemäß Anspruch 4 der Formel

$$X-(CH_2-CH_2O)_q-(CH_2)_r-SO_2-CH_2-CH_2-OH \quad (6),$$

worin

X = Cl, Br, $OCO-(CH_2)_{0-3}-CH_3$, $O-CO-C_6H_5$,
q = 0 oder 1,
r = 3 oder 4, wenn q = 0 und
r = 2, 3 oder 4, wenn q = 1

dadurch gekennzeichnet, daß man Thioether der Formel

$$X-(CH_2-CH_2-O)_q-(CH_2)_r-S-CH_2-CH_2-OH$$

wonn
q und rdie oben angegebenen Bedeutung haben,
in wässriger oder wässrig-organischer Lösung, vorzugsweise mit Wasserstoffperoxid unter Katalyse von Wolfram-

oder Vanadiumverbindungen, oxidiert.

6. Verfahren zur Herstellung von Reaktivfarbstoffen mit einem faserreaktiven Rest der Formel $SO_2Z$, worin Z die in Anspruch 1 angegebene Bedeutung hat, dadurch gekennzeichnet, daß man in der Synthese eine gemäß Anspruch 1 oder 2 hergestellte Verbindung einsetzt.

## Claims

1. Process for the preparation of compounds of the formula

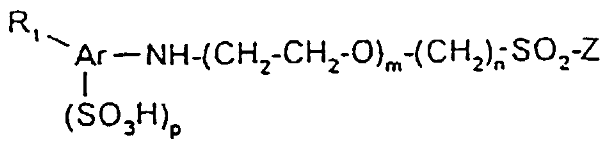

(1)

in which

Ar = phenyl or naphthyl radical,
$R_1$ = H, $C_1$-$C_4$-alkyl, Cl, Br, $C_1$-$C_4$-alkoxy or COOH, acylamino, in particular acetamino, $SO_2$-alkylene-OH,
m = 0 or 1
n = 2, 3 or 4,
p = 0-2,
Z = $-CH=CH_2$, $-CH_2-CH_2-OSO_3H$, $-CH_2-CH_2-S_2O_3H$, $-CH_2-CH_2-OCOCH_3$, $CH_2-CH_2-OPO_3H_2$, $-CH_2-CH_2-OH$,

characterized in that the radical X in sulphones of the formula

$$X-(CH_2-CH_2-O)_m-(CH_2)_n-SO_2-CH_2-CH_2OH \quad (2)$$

in which

X = Cl, Br, $OCO-(CH_2)_{0-3}-CH_3$, $O-CO-C_6H_5$,

is replaced by

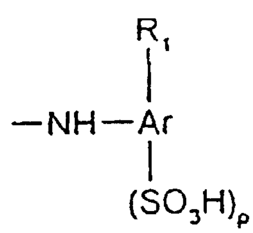

2. Process according to Claim 1, characterized in that a compound of the formula

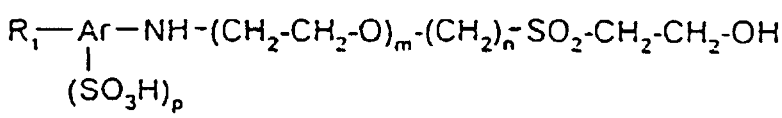

(5)

in which
$R_1$, p, m and n as defined in Claim 1, is converted in a conventional manner by functionalization of the OH group

into compounds in which X = $-CH{=}CH_2$, $-CH_2-CH_2-OSO_3H$, $-CH_2-CH_2-S_2O_3H$, $-CH_2-CH_2-OCOCH_3$, $CH_2-CH_2-OPO_3H_2$, and, if p = 0, $SO_3H$ groups are furthermore, if desired, introduced into the aromatic radical.

**3.** Process according to Claim 1, characterized in that, a sulphone of the formula (2), 3-chloropropyl 2'-hydroxyethyl sulphone is reacted with aniline to give a compound of the formula

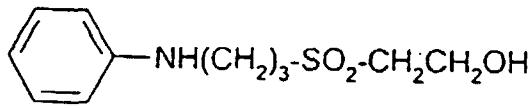

**4.** Sulphones of the formula

$$X-(CH_2-CH_2-O)_q-(CH_2)_r-SO_2-CH_2-CH_2-OH \quad (6)$$

in which

X = Cl, Br, $OCO-(CH_2)_{0\text{-}3}-CH_3$, $O-CO-C_6H_5$,
q = 0 or 1,
r = 2,3 or 4 if q = 1 and
r = 3 or 4 if q = 0.

**5.** Process for the preparation of sulphones according to Claim 4 of the formula

$$X-(CH_2-CH_2-O)_q-(CH_2)_r-SO_2-CH_2-CH_2-OH \quad (6)$$

in which

X = Cl, Br, $OCO-(CH_2)_{0\text{-}3}-CH_3$, $O-CO-C_6H_5$,
q = 0 or 1,
r = 3 or 4 if q = 0 and
r = 2, 3 or 4 if q = 1,

characterized in that thioethers of the formula

$$X-(CH_2-CH_2-O)_q-(CH_2)_r-S-CH_2-CH_2-OH$$

in which
q and r are as defined above,
are oxidized in aqueous or aqueous-organic solution, preferably using hydrogen peroxide with catalysis by compounds of tungsten or vanadium.

**6.** Process for the preparation of dyes containing a fibre-reactive reactive radical of the formula $SO_2Z$, in which Z is as defined in Claim 1, characterized in that a compound prepared according to Claim 1 or 2 is employed in the synthesis.

## Revendications

**1.** Procédé de préparation de composés de formule

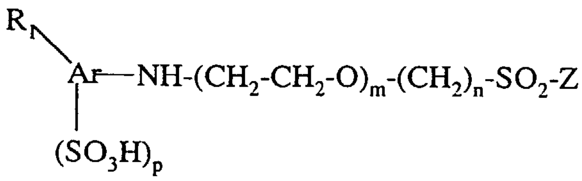

(1)

dans laquelle

Ar = groupe phényle ou naphtyle,
$R_1$ = H, alkyle en $C_1$-$C_4$, Cl, Br, alcoxy en $C_1$-$C_4$ ou COOH, acylamino, plus particulièrement acétamino, $SO_2$-alkylène-OH,
m = 0 ou 1,
n = 2, 3 ou 4,
p = 0 à 2,
Z = $-CH=CH_2$, $-CH_2-CH_2-OSO_3H$, $-CH_2-CH_2-S_2O_3H$, $-CH_2-CH_2-OCOCH_3$, $CH_2-CH_2-OPO_3H_2$, $-CH_2-CH_2-OH$,

caractérisé en ce que, dans des sulfones de formule

$$X-(CH_2-CH_2-O)_m-(CH_2)_n-SO_2-CH_2-CH_2OH \quad (2)$$

dans laquelle

X = Cl, Br, $OCO-(CH_2)_{0-3}-CH_3$, $O-CO-C_6H_5$,

on échange le groupe X contre le groupe

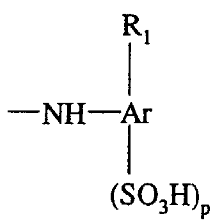

2. Procédé selon la revendication 1, caractérisé en ce que l'on convertit de la manière habituelle, par une fonctionnalisation du groupe OH, un composé de formule

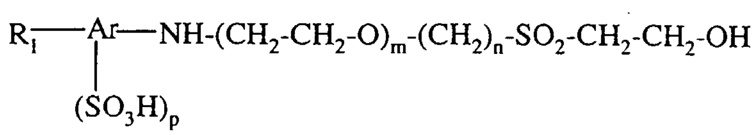

(5)

dans laquelle
$R_1$, p, m et n ont les significations indiquées dans la revendication 1, en composés pour lesquels Z = $-CH=CH_2$, $-CH_2-CH_2-OSO_3H$, $-CH_2-CH_2-S_2O_3H$, $-CH_2-CH_2-OCOCH_3$, $CH_2-CH_2-OPO_3H_2$, et, lorsque p = 0, on introduit encore le cas échéant des groupes $SO_3H$ dans le radical aromatique.

3. Procédé selon la revendication 1, caractérisé en ce que l'on convertit la 3-chloropropyl-2'-hydroxyéthylsulfone, sulfone de formule (2), par réaction avec l'aniline, en un composé de formule

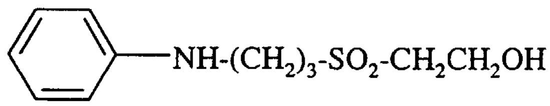

**4.** Sulfones de formule

$$X\text{-}(CH_2\text{-}CH_2\text{-}O)_q\text{-}(CH_2)_r\text{-}SO_2\text{-}CH_2\text{-}CH_2\text{-}OH \quad (6)$$

dans laquelle

X = Cl, Br, $OCO\text{-}(CH_2)_{0\text{-}3}\text{-}CH_3$, $O\text{-}CO\text{-}C_6H_5$,
q = 0 ou 1,
r = 2, 3 ou 4 lorsque q = 1,
r = 3 ou 4 lorsque q = 0.

**5.** Procédé de préparation des sulfones selon la revendication 4, répondant à la formule

$$X\text{-}(CH_2\text{-}CH_2\text{-}O)_q\text{-}(CH_2)_r\text{-}SO_2\text{-}CH_2\text{-}CH_2\text{-}OH \quad (6)$$

dans laquelle

X = Cl, Br, $OCO\text{-}(CH_2)_{0\text{-}3}\text{-}CH_3$, $O\text{-}CO\text{-}C_6H_5$,
q = 0 ou 1,
r = 3 ou 4 lorsque q = 0
r = 2, 3 ou 4 lorsque q = 1,

caractérisé en ce que l'on oxyde des thioéthers de formule

$$X\text{-}(CH_2\text{-}CH_2\text{-}O)_q\text{-}(CH_2)_r\text{-}S\text{-}CH_2\text{-}CH_2\text{-}OH$$

dans laquelle
q et r ont les significations indiquées ci-dessus,
en solution aqueuse ou hydro-organique, de préférence à l'aide du peroxyde d'hydrogène et avec catalyse par des dérivés du tungstène ou du vanadium.

**6.** Procédé de préparation de colorants réactifs contenant un groupe réactif avec les fibres de formule $SO_2Z$, dans laquelle Z a les significations indiquées dans la revendication 1, caractérisé en ce que l'on met en oeuvre dans la synthèse un composé préparé selon la revendication 1 ou 2.